# EUROPEAN PATENT APPLICATION

(11) **EP 4 660 312 A1**
(43) Date of publication of application: **10.12.2025**
(21) Application number: 24840023.6
(22) Date of filing: 08.07.2024
(51) Int. Cl.: C12P 13/10, B01D 15/36, B01D 9/00

(54) **METHOD AND APPARATUS FOR PREPARING L-CITRULLINE**

(30) Priority: 11.07.2023 KR 20230089745
(71) Applicant: CJ CheilJedang Corporation, Seoul 04560 (KR)
(72) Inventor: HONG, Je-won, Seoul 04560 (KR); PARK, Shin-ae, Seoul 04560 (KR); KIM, Jungwon, Seoul 04560 (KR); YOO, Hyun-Woo, Seoul 04560 (KR); YU, Jaehun, Seoul 04560 (KR)
(74) Representative: Meissner Bolte Partnerschaft mbB
(86) International application number: PCT/KR2024/009634
(87) International publication number: WO 2025/014211

(57) **Abstract**

The present disclosure relates to a preparation method for L-citrulline, comprising: Step 1 of preparing a fermentation broth comprising L-citrulline; Step 2 of performing a continuous chromatography process on the fermentation broth to obtain a purified citrulline process liquid; and Step 3 of crystallizing and separating L-citrulline in the purified citrulline process liquid. According to the present disclosure, high purity L-citrulline crystals can be obtained in high yield from the fermentation broth.

## Description

### [Technical Field]

The present disclosure relates to a preparation method and device for obtaining high-purity L-citrulline crystals in high yield.

### [Background Art]

Food grade citrulline is usually manufactured synthetically or through a fermentation process. When manufacturing citrulline through a fermentation process, a process of removing impurities such as fermentation byproducts and crystallization are necessary to obtain high-purity citrulline. Several purification processes are required to obtain high-purity citrulline through a fermentation process; however, an increase in the number of purification steps leads to the problem of reduced recovery yield of citrulline.

As a process for obtaining high-purity citrulline from a fermentation broth, purification methods such as crystallization or adsorption using resin columns are mainly employed.

For example, CN 105039216 B discloses obtaining citrulline by converting citrulline from an arginine fermentation broth, passing the obtained citrulline through a cation chromatography resin, and crystallizing citrulline by adding alcohol. This process employs a chromatography resin but also uses alcohol, an organic solvent, and thus is a process involving the use of chemical substances.

In the case of previously developed prior art, chemical substances must be used in either the process using resin columns or the crystallization step, which is unfavorable from an ESG perspective. Additionally, such processes fail to yield citrulline crystals with a purity of 98.5%, which is the specification required for food-grade use. In addition, as more steps are added to the process, the amount of citrulline obtained relative to the amount of citrulline in the initial fermentation broth decreases, and when impurities are not adequately removed at each step, product having a purity of 98.5% or more cannot be obtained, thereby failing to meet the specification required for food use.

Accordingly, there is a need to develop a process capable of producing food-grade high-purity L-citrulline at high yield without the use of chemical substances such as organic solvents.

### [Prior Art Documents]

### [Patent Documents]

(Patent Document 1) CN 105039216 B

### [Disclosure]

### [Technical Problem]

It is one object of the present disclosure to provide, for a process of producing L-citrulline using a fermentation process, a technique for purifying high-purity L-citrulline without the use of organic solvents.

It is another object of the present disclosure to provide a technique for obtaining high-purity L-citrulline at high yield while reducing the number of steps in the purification process.

### [Technical Solution]

In order to achieve the above objects, the present disclosure provides, for a process of producing L-citrulline using a fermentation process, a technique for purifying high-purity L-citrulline without the use of organic solvents.

In addition, the present disclosure provides a technique for obtaining high-purity L-citrulline at high yield while reducing the number of steps in the purification process.

### [Advantageous Effects]

The preparation method for L-citrulline according to an embodiment of the present disclosure allows removal of impurities from a fermentation broth without the use of a chemical solvent by using a continuous chromatography process, and thus is environmentally friendly. In addition, after the removal of impurities, the L-citrulline crystals can be easily separated from the process liquid by converting the crystal form of L-citrulline through concentration and cooling crystallization. L-citrulline crystals separated through such a method have high purity and low moisture content, and therefore, the energy required for drying can be reduced.

### [Brief Description of the Drawings]

FIG. 1 is a flow chart illustrating a preparation method for L-citrulline according to the present disclosure.
FIG. 2 is a flow chart illustrating a preparation method for L-citrulline according to an embodiment of the present disclosure.
FIG. 3 is a block diagram illustrating a continuous chromatography unit in a preparation system for L-citrulline according to an embodiment of the present disclosure
FIG. 4 is a graph illustrating the separation pattern of citrulline and impurities using a chromatography resin column.
FIG. 5 is an image analyzing various crystal forms of L-citrulline.

### [Detailed Description of Preferred Embodiments]

The present disclosure will be described in detail as follows. Meanwhile, each description and embodiment described herein can be applied to other descriptions and embodiments, respectively. That is, all combinations of various elements described herein fall within the scope of the present disclosure. Further, the scope of the present disclosure is not limited by the specific description described below.

The present disclosure relates to a process for obtaining L-citrulline from a fermentation broth using continuous chromatography. According to the present disclosure, the use of continuous chromatography can simplify the purification process, thereby enabling L-citrulline to be obtained at high yield. In addition, by avoiding the use of an organic solvent during the performance of a purification process involving continuous chromatography, high-purity L-citrulline can be obtained.

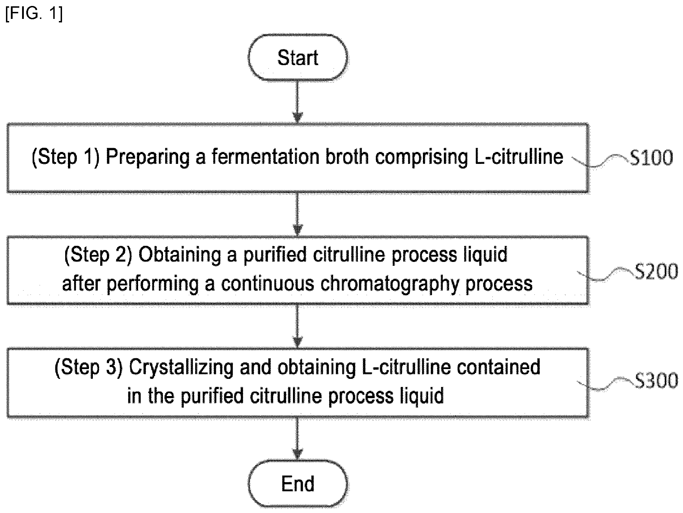

FIG. 1 is a flow chart illustrating a preparation method for L-citrulline according to the present disclosure.

Referring to FIG. 1, a preparation method for L-citrulline according to the present disclosure comprises: Step 1 (S100) of preparing a fermentation broth comprising L-citrulline; Step 2 (S200) of performing a continuous chromatography process on the fermentation broth to obtain a purified citrulline process liquid; and Step 3 (S300) of crystallizing and separating L-citrulline in the purified citrulline process liquid. Hereinafter, each step is described in detail.

Step 1 (S100) is a step of preparing a fermentation broth comprising L-citrulline. As used herein, L-citrulline, which is represented by the chemical formula H₂NC(O)NH(CH₂)₃CH(NH₂)CO₂H, is referred to as 2-amino-5-(carbamoylamino)pentanoic acid under IUPAC nomenclature. L-citrulline is known as a major metabolite in the urea cycle.

As used herein, the term "fermented product" may refer to a product formed by enzymatic or metabolic decomposition of organic substances using microorganisms. For example, a fermented product may comprise the culture itself obtained by culturing microorganisms in a culture medium, or a concentrate, dried product, or freeze-dried product of the culture obtained after removing the microbial strain therefrom. Further, in this case, the fermentation broth may comprise the entire fermented product comprising L-citrulline, or may be a fermented product comprising L-citrulline from which impurities have been removed.

The "microorganism producing L-citrulline" or "microorganism producing L-citrulline or a desired product" used in Step 1 (S100) comprises both wild-type microorganisms and microorganisms that have undergone natural or artificial genetic modification. It may be a microorganism that has undergone genetic modification for the production of a desired polypeptide or L-citrulline, in which a specific mechanism is weakened or enhanced due to factors such as the insertion of exogenous genes or the enhancement or inactivation of endogenous gene activity.

The microorganism producing L-citrulline of the present disclosure may be a microorganism that naturally possesses L-citrulline producing ability or a microorganism in which L-citrulline producing ability has been imparted to a parent strain lacking L-citrulline producing ability, but is not limited thereto. Specifically, in the present disclosure, the microorganism producing L-citrulline or a desired product, or the microorganism that exhibits the ability to produce L-citrulline or a desired product, may be a microorganism in which some genes in the biosynthetic pathway for a desired protein or desired product are enhanced or attenuated or a microorganism in which some genes in the degradation pathway for a desired protein or desired product are enhanced or attenuated. "Enhancing" or "increasing" the L-citrulline producing ability of a microorganism of the present disclosure means the L-citrulline producing ability of the microorganism of the present disclosure is improved compared to that of a microorganism other than the microorganism of the present disclosure, a parent strain, or a non-modified microorganism. For example, the microorganism of the present disclosure may have an L-citrulline producing ability that is improved by about 1% or more, 10% or more, 100% or more, 200% or more, 500% or more, 1000% or more, 1100% or more, 1200% or more, or 1300% or more, compared to the L-citrulline productivity of another microorganism, and may have an L-citrulline producing ability that is improved by about 1.01 times or more, 2 times or more, 5 times or more, 10 times or more, 11 times or more, 12 times or more, or 13 times or more, but is not limited thereto. The term "about" refers to a range encompassing ±0.5, ±0.4, ±0.3, ±0.2, ±0.1, etc., including all numerical values in a range equal to or similar to the numerical value following the term "about", but the range is not limited thereto.

The above-described microorganism used in Step 1 (S100) may be at least one microorganism selected from the group consisting of the yeast *Candida famata,* the ascomycetes *Eremothecium ashbyii* and *Ashbya gossypii,* and the bacteria *Bacillus subtilis* and *Corynebacterium* sp.

When the microorganism used in Step 1 (S100) is a *Corynebacterium sp.,* the microorganism may specifically be *Corynebacterium glutamicum, Corynebacterium crudilactis, Corynebacterium deserti, Corynebacterium efficiens, Corynebacterium callunae, Corynebacterium stationis, Corynebacterium singulare, Corynebacterium halotolerans, Corynebacterium striatum, Corynebacterium ammoniagenes, Corynebacterium pollutisoli, Corynebacterium imitans, Corynebacterium testudinoris, Corynebacterium crenatum,* or *Corynebacterium flavescens,* or more specifically *Corynebacterium glutamicum,* but is not limited thereto.

Step 1 (S100) may further comprise culturing a "microorganism producing L-citrulline". The culturing of the microorganism may be performed in a suitable medium known in the art under suitable culturing conditions known in the art. Such a culturing process may be readily adjusted and used by a person skilled in the art according to the selected strain. Specifically, the culturing may be batch culturing, continuous culturing, and fed-batch culturing, but is not limited thereto. As used herein, the term "medium" refers to a substance in which nutrients required to culture the microorganism are mixed as a main component, and the medium supplies nutrients, growth factors, *etc.,* including water, which are indispensable for survival and development. Specifically, as long as the medium is used for the common culture of microorganisms, any medium and culture conditions may be used as the medium and culture conditions for culturing the microorganism of the present disclosure without particular limitation. The microorganism of the present disclosure may be cultured in a common medium containing suitable carbon sources, nitrogen sources, phosphorus sources, inorganic compounds, amino acids, and/or vitamins, *etc.,* while controlling the temperature, pH, *etc.* under aerobic conditions.

The fermentation broth prepared in Step 1 (S100) is subsequently introduced into a continuous chromatography process.

In Step 2 (S200), a continuous chromatography process is performed on the fermentation broth to obtain a purified citrulline process liquid.

"Continuous chromatography" refers to a process in which a conventional batch chromatography process has been developed into a continuous process. Specifically, in a chromatography device, a solid phase and a liquid phase can be continuously supplied, and as the solid phase and the liquid phase move in opposite directions to each other, countercurrent contact is induced, thereby enabling more efficient separation of substances. As used herein, continuous chromatography may be used as a concept encompassing true moving bed (TMB) chromatography and simulated moving bed (SMB) chromatography. Further, since both TMB chromatography and SMB chromatography have the same principle, a person skilled in the art may appropriately select and use them by considering productivity and other factors.

Continuous chromatography may comprise a plurality of resin columns, and a strong acid cation exchange resin may be provided as a stationary phase in the resin columns. The functional group of the strong acid cation exchange resin may be a sulfuric acid group, but is not limited thereto. Further, the base material of the strong acid cation exchange resin used herein is not limited as long as it can be adsorbed to the strong acid functional group. For example, styrene-divinyl benzene copolymers may be used, but is not limited thereto. As a specific example, the strong acid cation exchange resin may be a sulfonated styrene-divinylbenzene copolymer, but is not limited thereto.

Compared to single-column chromatography that includes only one resin column, continuous chromatography exhibits superior resolution between L-citrulline and impurities. Thus, the section in which L-citrulline and impurities are co-eluted is significantly reduced, and high-purity L-citrulline can be obtained in most of the section in which L-citrulline is eluted. Accordingly, both the purity and recovery of L-citrulline can be increased. In contrast, in the case of single-column chromatography, the resolution is relatively low, and thus there exists a section where L-citrulline and impurities are co-eluted. In order to obtain high-purity L-citrulline, L-citrulline must be obtained only from the section in which impurities are not eluted and not from the section in which L-citrulline and impurities are co-eluted. Therefore, a significant amount of L-citrulline is discarded along with impurities, leading to a decrease in L-citrulline recovery.

In Step 2 (S200), as the fermentation broth passes through the continuous chromatography resin columns, ions, organic acids, other amino acids, residual sugars, *etc.* that may be in the fermentation broth in addition to L-citrulline may be removed.

Specifically, Step 2 (S200) may further comprise Step 2-1 of introducing the fermentation broth into a plurality of resin columns to separate L-citrulline from impurities, and Step 2-2 of introducing water into the resin columns in which L-citrulline has been retained to obtain a purified citrulline process liquid comprising L-citrulline and water.

Continuous chromatography may comprise a plurality of resin columns and a strong acid cation exchange resin provided in the resin columns. In Step 2-1, the fermentation broth may pass through the above-described resin columns and come into contact with the strong acid cation exchange resin provided therein, whereby impurities in the fermentation broth may be adsorbed to and removed by the strong acid cation exchange resin.

Next, water, which is not an organic solvent, may be introduced into the resin columns in which L-citrulline is retained to obtain a purified citrulline process liquid comprising L-citrulline and water. The purified citrulline process liquid may refer to a solution from which impurities such as ions, organic acids, other amino acids, residual sugars, *etc.* originally in the fermentation broth have been removed as described above, and in which the concentration of L-citrulline has increased.

In Step 2 (S200), as described above, L-citrulline is not adsorbed to the resin, whereas impurities are adsorbed to the resin and thereby removed. Accordingly, there is no need for a separate eluent or regenerant to separate L-citrulline from the resin, making the process environmentally friendly. Additionally, since the continuous chromatography process does not require an adsorption/elution process, the hourly productivity is higher, and the amount of process water used can be reduced compared to the ion exchange process. In conventional techniques, in order to purify a fermentation broth comprising L-citrulline, L-citrulline is adsorbed onto an ion exchange resin and then separated using a solvent such as ammonia or alcohol. These technologies are disadvantageous from an ESG perspective because they utilize chemical substances.

Then, the purified citrulline process liquid obtained in Step 2 (S200) is introduced into a crystallization and separation process.

Step 3 (S300) crystallizes and thereby separates L-citrulline in the purified citrulline process liquid. As used herein, "crystallization" refers to a phenomenon in which a liquid or amorphous solid forms a crystal, and may occur accompanied by two phenomena: generation and growth of crystal nuclei.

In Step 3 (S300), through concentration of the purified citrulline process liquid and the crystal form conversion of the L-citrulline crystals, L-citrulline crystals can be obtained that allow for easy separation of the mother liquor and contain a small amount of moisture. Further details on Step 3 (S300) are described below.

According to an embodiment of the present disclosure, continuous chromatography and crystallization processes are used to prepare an L-citrulline product from a fermentation broth. The use of a continuous chromatography process allows impurities to be efficiently removed from a fermentation broth without a complicated multistep process and it does not require the use of a chemical solvent, thereby making the process environmentally friendly. Additionally, through concentration of the purified citrulline process liquid and crystal form conversion of the L-citrulline crystals, the crystallization process allows L-citrulline crystals to be obtained that allow for easy separation of the mother liquor and contain a small amount of moisture. According to the present disclosure, high-purity L-citrulline crystals can be obtained in high yield in an environmentally friendly manner.

Hereinabove, the preparation method for L-citrulline of the present disclosure has been described. Hereinafter, the detailed processes of a preparation method for L-citrulline according to an embodiment of the present disclosure are described in further detail.

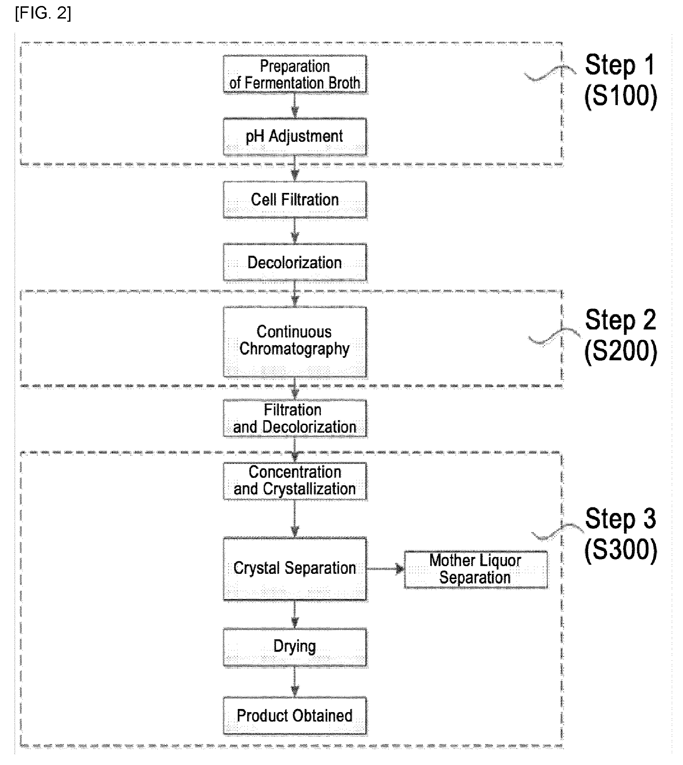

FIG. 2 is a flow chart illustrating a preparation method for L-citrulline according to an embodiment of the present disclosure.

In the following description of FIG. 2, explanations of processes identical to those described above will be omitted to avoid redundancy.

Referring to FIG. 2, Step 1 (S100) of preparing a fermentation broth comprising L-citrulline comprises preparing a fermentation solution and adjusting pH. In the pH adjustment step, the pH of the prepared fermentation broth may be adjusted to a range of 2 to 7. Generally, the pH of the fermentation broth may be a pH of 6 to 7, and an acidic solution such as HCl or H₂SO₄ may be added to adjust the pH of the fermentation broth. However, the substance that may be used to adjust the pH of the fermentation broth can be appropriately selected as long as it does not affect L-citrulline. Adjusting the pH of the fermentation broth to the above-described range enables the implementation of a high-efficiency cell separation process through enhanced cell aggregation, thereby improving the effectiveness of the subsequent decolorization process.

After performing Step 1 (S100), a process of removing the cells from the fermentation broth may additionally be performed. For example, before or after performing Step 2 (S200) and/or Step 3 (S300), the fermentation broth can be filtered through a membrane that does not allow cells to pass through, thereby removing the cells from the fermentation broth.

Additionally, a decolorization process may be performed after performing Step 1 (S100). The decolorization process is intended to remove residual coloring agents from the fermentation broth and may be performed by an adsorption decolorization method using activated carbon or clay (activated clay, acid clay) as an adsorbent. However, the above description is exemplary, and the method for performing the decolorization process can be appropriately selected by considering the properties of the fermentation broth comprising L-citrulline, the characteristics of subsequent processes, *etc.*

Meanwhile, the drawing illustrates that pH adjustment, cell filtration, and decolorization are performed sequentially after preparation of the fermentation broth, but the order of pH adjustment, cell filtration, and decolorization processes may be changed as needed. For example, the pH may be adjusted after completing the cell filtration and decolorization steps, or the decolorization step may be performed before the cell filtration step.

Next, after performing Step 2 (S200), which performs a continuous chromatography process on the fermentation broth to obtain a purified citrulline process liquid, filtration and decolorization processes may be additionally performed. Through the filtration and decolorization processes, any impurities that may remain in the purified citrulline process liquid can additionally be removed. The filtration and decolorization processes may be performed in a conventional manner similar to that described above for Step 1 (S100) and may be omitted if necessary to simplify the process.

Subsequently, Step 3, which crystallizes and thereby separates L-citrulline in the purified citrulline process liquid, is performed. Step 3 (S300) may specifically include a concentration and crystallization step, a crystal separation step, and a drying step.

The concentration and crystallization step of Step 3 (S300) may further comprise concentrating the purified citrulline process liquid prior to crystallizing L-citrulline. The concentration of the purified citrulline process liquid may be performed in a conventional concentrator (e.g., a forced circulation concentrator, a thin film concentrator, or a rotary concentrator) selected by a person skilled in the art. The concentration of L-citrulline in the process liquid after concentration may be about 100 g/L to less than about 800 g/L, about 200 g/L to less than about 700 g/L, about 300 g/L to less than about 600 g/L, about 100 g/L to less than about 500 g/L, about 200 g/L to less than about 500 g/L, about 300 g/L to less than about 500 g/L, and more specifically, about 400 g/L to about 500 g/L, but is not limited thereto. The recovery rate of citrulline can be increased by increasing the concentration of the purified citrulline process liquid. However, if the concentration of L-citrulline in the process liquid after concentration exceeds about 500 g/L, an excessive amount of crystals forms, making stirring difficult. Accordingly, the concentration process may be performed so that the concentration of L-citrulline in the process liquid after concentration is less than about 500 g/L.

The above-described concentration process in Step 3 (S300) may be performed at a temperature range of about 50°C to about 90°C. In some cases, the concentration process may be performed at a temperature in the range of about 50°C to about 80°C, about 50°C to about 70°C, about 60°C to about 80°C, about 60°C to about 70°C, or about 70°C to about 80°C. During the concentration process, L-citrulline in the purified citrulline process liquid may form L-citrulline crystals. The L-citrulline crystals formed during the concentration process may be amorphous. Since amorphous crystals are difficult to separate from the mother liquor of the process liquid, it is difficult to obtain clean crystals. Accordingly, Step 3 may further comprise converting the amorphous crystalline form of the L-citrulline crystals to an anhydrous crystal form through a cooling process after the concentration of the purified citrulline process liquid. When performing the concentration process within the temperature range described above, the crystalline form of the L-citrulline crystals may change. Specifically, by performing a concentration process in the temperature range described above and subsequently performing a cooling crystallization process, L-citrulline crystals may form in a form that is easy to separate from the mother liquor. The L-citrulline crystals formed after the cooling crystallization process may be small, rectangular, anhydrous crystals.

The cooling crystallization process can be performed by cooling the temperature of the concentrated process liquid comprising L-citrulline crystals to a range of about 5°C to about 45°C. In some cases, the cooling temperature may be from about 5°C to about 40°C, about 10°C to about 35°C, about 15°C to about 30°C, about 20°C to about 25°C, about 5°C to about 35°C, or about 10°C to about 30°C. In the cooling crystallization process, the temperature of the process liquid may be gradually lowered so that all amorphous L-citrulline crystals may be converted to an anhydrous crystalline form. For example, the cooling rate may be from about 3°C/h to about 7°C/h. The cooling rate may be determined by considering process factors such as the temperature of the concentrated process liquid and the amount of L-citrulline.

The anhydrous crystals of L-citrulline produced after the cooling crystallization process have a relatively uniform shape and are easy to separate from the mother liquor of the process liquid. Accordingly, the separated L-citrulline crystals may have a relatively low moisture content compared to amorphous crystals. L-citrulline crystals with low moisture content allow for quality stabilization as there are no issues with dehydration or washing during crystal separation. In addition, the use of a dryer can eliminate risks associated with transfer and flow, and reduce the amount generated during the drying process, thereby minimizing loss. In addition, there is an advantage in that the process economy can be improved by reducing the amount of dryer steam used as the wet moisture content decreases.

Separation of L-citrulline crystals and mother liquor may be performed using a liquid-solid separator such as a pressure reducing membrane filtration device, a pressure increasing membrane filtration device, or a centrifugal separator, but is not limited thereto. The mother liquor remaining after obtaining the L-citrulline crystals may be reintroduced into Step 1 (S100) or Step 2 (S200) to improve the final purification recovery rate of L-citrulline.

Next, the obtained L-citrulline crystals can be washed and dried to prepare the final product, L-citrulline crystals. Washing and drying can be performed by any of the commonly used methods.

As described above, the preparation process for L-citrulline according to an embodiment of the present disclosure allows for converting the form of L-citrulline crystals in the concentration and crystallization processes, thereby enabling easy separation from the mother liquor and obtaining L-citrulline crystals having low moisture content. Therefore, by using the above preparation method, high-purity L-citrulline crystals can be obtained, and the energy required for the process can also be reduced.

### [Mode for Carrying Out the Invention]

The present disclosure will be described in detail by way of Examples. However, these Examples are given for illustrative purposes only, and the scope of the invention is not intended to be limited by these Examples. Meanwhile, the technical descriptions absent in the present disclosure may be sufficiently understood and readily practiced by a person of ordinary skill in the art of the present disclosure or related art.

Hereinafter, a preparation process and preparation system for L-citrulline according to an embodiment of the present disclosure are described. Hereinafter, the advantageous effects referred to in the present disclosure will be described with reference to the experimental results of the Examples and Comparative Examples.

### [Review of Experimental Method]

The analysis of the experimental results described in the present disclosure was performed in the following manner.

### (1) HPLC Method for Quantitative Analysis of L-citrulline

In the present disclosure, the HPLC analysis conditions for analyzing the purity and concentration of L-citrulline are as follows:
Equipment: HPLC 1260 Infinity System (Agilent Technology Inc.)
Column: HP C18 (150 mm × 3.9 mm; 5 µm)
Mobile phase: 25 mM KH₂PO₄, 12.2 mM OSA in Water
Flow rate: 1 mL/min
Temperature: 40°C
Detection: UV at 210 nm
Injection volume: 5 µL

### (2) Method for Measuring the Purity of L-citrulline Crystals

In the present disclosure, the quality of L-citrulline crystals is evaluated based on the purity of L-citrulline, and the method follows the following order:
(a) preparing L-citrulline crystals by placing them in a vacuum desiccator containing silica gel at a vacuum level of 20 mmHg or less for 24 hours to remove residual moisture, and cooling the temperature of the L-citrulline crystals to room temperature;
(b) weighing 0.5000 g of the L-citrulline crystals cooled to room temperature, placing the crystals in a 1 L volumetric flask, and diluting with triple-distilled water to prepare a sample at a concentration of 0.5000 g/L;
(c) first treating L-citrulline standard crystals (Sigma, ≥ 99.0%) in the same manner as in step (a) and then weighing 0.5000 g of the crystals, placing the crystals in a 1 L volumetric flask, and diluting with triple-distilled water to prepare a sample at a concentration of 0.5000 g/L, then confirming the purity of the standard product based on the certificate of analysis provided by the manufacturer of the standard reagent, and converting the concentration of L-citrulline in the sample ([converted L-citrulline concentration] = 0.5000 g/L × [purity of the standard product]); and
(d) analyzing the sample prepared in step (b) using HPLC, with the sample prepared in step (c) serving as an external standard, to determine the purity of the L-citrulline crystals used in step (a).

### (3) Method for Analyzing the Content of L-citrulline Based on Solids in the Fermentation Broth or Eluate Obtained From the Chromatography Process

In the present disclosure, the quality of the fermentation broth or an eluate obtained from the chromatography process is evaluated based on the content of L-citrulline in the solids obtained by removing moisture from the solution, and the method follows the following order:
(a) placing approximately 5 g of sea sand (10 to 20 mesh; Daejung Chemicals & Metals Co., Ltd.) in a ceramic container after deodorization, placing the container in a forced convection oven at 105°C for 3 hours to remove residual moisture, and then placing it in a vacuum desiccator containing silica gel for 1 hour to cool to room temperature;
(b) adding the solution to be analyzed into the container from step (a), and quantifying the [mass of the solution] added by measuring the difference in weight before and after the addition;
(c) placing the container from step (b) in a forced convection oven at 105°C for 3 hours to remove residual moisture, then placing it in a vacuum desiccator containing silica gel for 1 hour to cool to room temperature, then quantifying the amount of moisture removed based on the weight difference, and calculating the content based on the mass of solids in the solution to be measured ([solid content by mass] = ([mass of the solution] - [mass of removed moisture]) / [mass of the solution]);
(d) measuring the density of the solution to be analyzed using a hydrometer;
(e) measuring the concentration of L-citrulline in the solution to be analyzed using HPLC; and
(f) converting the content of L-citrulline in the solids obtained by removing moisture from the solution to be analyzed using the content based on the mass of the solids in the solution, the density, and the concentration of L-citrulline ([content of L-citrulline in the solids obtained by removing moisture from the solution] = [concentration of L-citrulline] / [density of the solution] / [solid content by mass of the solution]).

### Experimental Example 1. Separation Process of L-citrulline From Fermentation Broth

In Experimental Example 1, in performing a process for separating L-citrulline from a fermentation broth, a comparison was made between the result obtained using continuous chromatography according to an embodiment of the present disclosure (Example 1), and the results obtained using a cation exchange resin according to the conventional technique (Comparative Example 1) or using a single-column chromatography resin (Comparative Example 2).

### Preparation Example. Preparation of Fermentation Broth Comprising L-citrulline

A fermentation broth comprising L-citrulline was obtained by culturing an L-citrulline producing microorganism in a fermentation medium. The pH of the prepared L-citrulline fermentation broth was 7.0, and the concentration of L-citrulline was 130 g/L. The content of L-citrulline in the solids obtained by removing moisture from the L-citrulline fermentation broth was 70%. The pH of the fermentation broth was adjusted to pH 4.0 using 98% sulfuric acid. The fermentation broth was subjected to cell separation using a ceramic membrane filter system from TAMI Co., applying a membrane with a pore size of 0.14 µm, thereby obtaining a filtrate. After cell separation, the L-citrulline fermentation broth was prepared as a feed solution for the following SMB chromatography.

### Example 1. Separation of Citrulline Using Continuous Chromatography

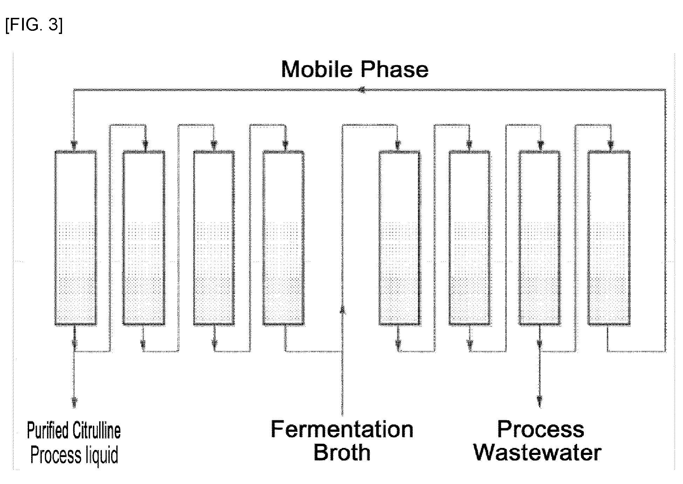

In order to obtain an eluate in which L-citrulline is separated from the fermentation broth comprising L-citrulline prepared in the Preparation Example, an SMB chromatography device (NOVASEP) with a resin (TRILITE MCK32L) with a strong acid functional group as a fixed phase was used. A schematic diagram of the SMB chromatography device is shown in FIG. 3.

The SMB chromatography device consists of a total of eight resin columns as shown in FIG. 3. Each column has a volume of 7.0 L, and the resin is filled to 95% of the column volume. The SMB feed solution is injected into the fifth resin column at a flow rate of 4.5 L/hr. Mobile phase (water) is injected into the first resin column at a flow rate of 18.2 L/hr. The SMB product process liquid (eluate) is discharged from the first resin column at a flow rate of 18.2 L/hr. The SMB process wastewater is discharged from the seventh resin column at a flow rate of 4.5 L/hr. Impurities that migrate into the process wastewater are mainly ions, organic acids, residual sugars, and non-citrulline amino acids. The resin columns were operated in a circulating manner, shifting in the direction from the first resin column to the eighth resin column every 25 minutes.

TRILITE MCK32L resin, a styrene-divinylbenzene copolymer resin with a sulfuric acid group, which is a strong acid, as a functional group, was loaded onto the resin columns of the chromatography device. 0.1 kL or more of the SMB chromatography feed solution was injected, and the device was operated to obtain the SMB product process liquid (eluate). The pH of the fermentation broth was adjusted to various values in the range of 2 to 7 using 98% sulfuric acid and 50% NaOH, and the recovery rate of the continuous chromatography process and the purity (%) of L-citrulline in the eluate obtained from the continuous chromatography process were measured. The recovery rate of the continuous chromatography process was calculated as the recovery rate of L-citrulline in the eluate compared to the fermentation broth introduced into the continuous chromatography device. The results are shown in Table 1 below.

**[Table 1]**

| **pH of fermentation broth** | **Recovery rate of continuous chromatography process (%)** | **Purity of L-citrulline eluate (%)** |
|---|---|---|
| 2.0 | 80.0 | 85 .3 |
| 2.5 | 80.9 | 86 .2 |
| 3.0 | 87.0 | 89 .3 |
| 3.5 | 88.5 | 90 .2 |
| 4.0 | 90.0 | 90 .1 |
| 4.5 | 91 .2 | 90 .0 |
| 5.0 | 90.8 | 90.0 |
| 5.5 | 90.6 | 90 .2 |
| 6.0 | 89.0 | 85 .5 |
| 6.5 | 85.0 | 83.0 |
| 7.0 | 83.0 | 82 .1 |

The yield of the continuous chromatography process was found to be 80% or more in the pH range of 2.0 to 7.0, 85% or more in the pH range of 3.0 to 6.5, and 90% in the pH range of 4.0 to 5.5. The purity of L-citrulline in the eluate obtained from the continuous chromatography process was found to be 85% or more in the pH range of 2.0 to 6.5 and 90% or more in the pH range of 3.5 to 5.5.

### Comparative Example 1. Separation of Citrulline Using Cation Exchange Resin

A fermentation broth was prepared as in the Preparation Example, and the pH of the filtrate was adjusted to a range of 0 to 2 using 98% sulfuric acid or 35% hydrochloric acid. A cation exchange resin (using TRILITE SCR-B) was regenerated using 35% hydrochloric acid and prepared in its H⁺ form. Then, the citrulline cell filtrate was introduced into the cation exchange resin, and water was added so that only citrulline in the filtrate was adsorbed onto the resin while the remaining impurities were removed. In order to obtain citrulline adsorbed onto the resin, the resin was treated with 35% hydrochloric acid so that H⁺ was adsorbed onto the resin and the previously adsorbed citrulline was eluted, thereby yielding a citrulline process liquid from which impurities were removed. The purity of the citrulline solution thus obtained was analyzed to be approximately 90%. In the case of Comparative Example 1, when a cation exchange resin was used, major impurities such as ions, other amino acids, and residual sugars could be removed from the fermentation broth; however, organic acids were difficult to remove because they are cationic at a pH of 2 or lower.

### Comparative Example 2. Separation of Citrulline Using a Single-Column Chromatography Resin

The filtrate prepared as a fermentation broth as in the Preparation Example was passed through a single chromatography resin column. TRILITE MCK32L resin, the same resin used in continuous chromatography, was used, the volume of the column was 520 mL, and more than 95% of the column was filled with resin. The process liquid was introduced at a flow rate of 0.5 L/hr, and mobile phase (water) was introduced at a flow rate of 1.5 L/hr. Citrulline, ions, organic acids, residual sugars, and other impurities in the citrulline fermentation broth were each separated by chromatography resin, size exclusion, ion adsorption, and adsorption by van der Waals force, and the like. Anions were first separated and eluted, followed by residual sugars, organic acids, and cations. Citrulline was eluted last. The pH of the fermentation broth was adjusted to various values in the range of 2 to 7 using 98% sulfuric acid and 50% NaOH, and the recovery rate of the single column chromatography process according to the pH of the fermentation broth and the purity (%) of L-citrulline in the eluate obtained from the single column chromatography process were measured. The recovery rate of the single column chromatography process was calculated as the recovery rate of L-citrulline separated from ions, residual sugars, organic acids, and other impurities compared to the fermentation broth introduced into the single column chromatography device. The results are shown in Table 2 below.

**[Table 2]**

| **pH of fermentation broth** | **Recovery rate of single column chromatography process (%)** | **Purity of L-citrulline eluate (%)** |
|---|---|---|
| 2.0 | 10.0 | 87.9 |
| 2.5 | 10 .1 | 88.9 |
| 3.0 | 10.7 | 89.8 |
| 3.5 | 12.8 | 90.2 |
| 4 .0 | 13.6 | 90.0 |
| 4.5 | 13.3 | 90.3 |
| 5.0 | 13.5 | 90.1 |
| 5.5 | 13.2 | 90.2 |
| 6.0 | 13.3 | 89.9 |
| 6.5 | 11.8 | 88.8 |
| 7.0 | 10.9 | 87.8 |

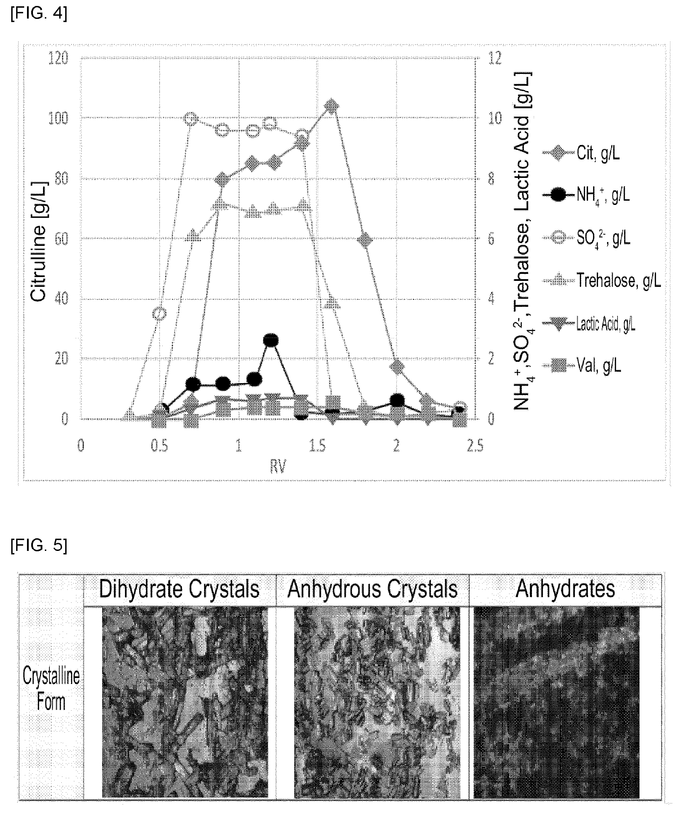

The purity of L-citrulline in the eluate obtained from the single column chromatography process, which is the method of Comparative Example 2, was found to be 87% or more in the pH range of 2.0 to 7.0 and 90% or more in the pH range of 3.5 to 5.5. However, as shown in FIG. 4, in the case of the yield of the single column chromatography process, the peaks of impurities and citrulline significantly overlap, and it was confirmed that the recovery rate of citrulline was markedly reduced to 10% or more in the pH range of 2.0 to 7.0 and to approximately 13% in the pH range of 4.0 to 6.0. Therefore, the comparison of Example 1 with Comparative Examples 1 and 2 confirmed that purification of the fermentation broth using continuous chromatography resulted in a higher recovery rate of the process liquid and higher purity of L-citrulline. In the separation process using the cation exchange resin of Comparative Example 1, it had the disadvantage that it was difficult to remove the organic acids and that an acid eluent was required. In addition, as in Comparative Example 2, when a single column is used instead of a continuous column for purifying the fermentation broth, the resolution is relatively low, so there exists a section where L-citrulline and impurities are co-eluted. In order to obtain high-purity L-citrulline, L-citrulline must be obtained only from the section in which impurities are not eluted and not from the section in which L-citrulline and impurities are co-eluted. Therefore, a significant amount of L-citrulline is discarded along with impurities, leading to a decrease in L-citrulline recovery.

However, when continuous chromatography is utilized as in Example 1 of the present disclosure, the resolution between L-citrulline and impurities is improved, so the section where L-citrulline and impurities are co-eluted is significantly reduced. Therefore, high-purity L-citrulline can be obtained in most of the sections where L-citrulline is eluted, thereby increasing both the purity and recovery rate of L-citrulline.

### Experimental Example 2. L-Citrulline Crystallization Process for Purified Citrulline Process liquid

In Experimental Example 2, the purified citrulline process liquid obtained according to Example 1 was subjected to concentration and crystallization, and the result obtained with temperature adjustment according to one embodiment of the present disclosure (Example 2) was compared with the result obtained without temperature adjustment (Comparative Example 3).

### Example 2. Concentration and Crystallization With Temperature Adjustment

First, decolorization of the purified citrulline process liquid obtained using resin for continuous chromatography was performed. For decolorization, activated carbon was added in an amount of 3% based on the weight of citrulline, and decolorization was performed at about 60°C for about 1 hour. After completion of the decolorization, the activated carbon was filtered out to obtain a decolorized process liquid. During the filtration, a glass filter (Merck) with a size of about 0.7 µm was used to completely remove the activated carbon. The decolorized process liquid was placed in a crystallization vessel (DAIHAN) and concentrated to form L-citrulline crystals. The concentration was performed at a high internal temperature in the range of about 50°C to about 90°C, and the solution was concentrated to about 500 g/L. Thereafter, the temperature was cooled to about 5°C to about 30°C at a rate of about 5°C per hour. As the temperature gradually decreased, small rectangular anhydrous crystals were formed from the initially amorphous crystals. The purity of the crystals thus obtained was 98.5% or more, and the moisture content was 5% or less. Through this process, crystals compliant with food-grade specification for citrulline content were obtained, and since the moisture content of the crystals was 5% or less, the cost of steam required for drying during the process could also be reduced. The results are shown in Table 3.

**[Table 3]**

| **Internal temperature during concentration (°C)** | **Cooling rate (°C/hr)** | **Cooling temperature (°C)** | **Moisture content (%)** | **Purity of L-citrulline crystals (%)** |
|---|---|---|---|---|
| 50 | 5 | 5 | 2.3 | 99.6 |
| 60 | 5 | 15 | 3.0 | 99.6 |
| 70 | 5 | 20 | 2.8 | 99.7 |
| 80 | 5 | 25 | 2.5 | 99.6 |
| 90 | 5 | 30 | 2.6 | 99.7 |

### Comparative Example 3. Concentration and Crystallization Without Temperature Adjustment

Decolorization of the purified citrulline process liquid obtained using resin for continuous chromatography was performed. For decolorization, activated carbon was added in an amount of 3% based on the weight of citrulline, and decolorization was performed at 60°C for 1 hour. After completion of the decolorization, the activated carbon was filtered out to obtain a decolorized process liquid. The purity of the process liquid was 97.5%. During the filtration, a glass filter (Merck) with a size of about 0.7 µm was used to completely remove the activated carbon. The decolorized process liquid was placed in a crystallization vessel (DAIHAN) and concentrated to form crystals. The solution was concentrated to 500 g/L at an internal temperature of 50°C or less, and the crystals were then separated. Unlike the example, no additional cooling was performed, and the crystals were amorphous with no discernible shape. Since the mother liquor could not be separated during the separation process, the purity of the crystals was 97.1%, and the moisture content was 22%. The results are shown in Table 4.

**[Table 4]**

| **Internal temperature during concentration (°C)** | **Moisture content (%)** | **Purity of L-citrulline crystals (%)** |
|---|---|---|
| 30 | 21 | 97 .1 |
| 40 | 22 | 97.0 |

As confirmed in FIG. 5, L-citrulline crystals may have various crystalline forms. The L-citrulline crystals obtained in Example 2 were found to have an anhydrous crystalline form, and the anhydrous crystals were found to have a relatively low moisture content of 5% or less. In contrast, the amorphous crystals of Comparative Example 3 were found to have a moisture content of 22%, which was significantly higher than that of the anhydrous crystals. In addition, the purity of the crystals was found to be lower in Comparative Example 3 than the purity of the anhydrous crystals prepared in Example 2. Therefore, it was confirmed that L-citrulline crystals having low moisture content and high purity could be prepared by the crystallization process according to one embodiment of the present disclosure.

As set forth above, a person skilled in the art will be able to understand that the present disclosure may be embodied in other specific forms without departing from the technical spirit or essential characteristics thereof. Therefore, the embodiments described above should be construed as being exemplified and not limiting the present disclosure. It should be understood that all changes or modifications derived from the definitions and the scopes of the claims and their equivalents fall within the scope of the present disclosure.

## Claims

1. A preparation method for L-citrulline, comprising:
Step 1 of preparing a fermentation broth comprising L-citrulline;
Step 2 of performing a continuous chromatography process on the fermentation broth to obtain a purified citrulline process liquid; and
Step 3 of crystallizing and separating L-citrulline in the purified citrulline process liquid.

2. The method according to claim 1, wherein Step 3 further comprises concentrating the purified citrulline process liquid.

3. The method according to claim 2, wherein Step 3 further comprises converting the amorphous crystal form of L-citrulline crystals into an anhydrous crystal form through a cooling process after the concentration of the purified citrulline process liquid.

4. The method according to claim 3, wherein the concentration of the purified citrulline process liquid is performed in the range of 50°C to 90°C, and the cooling process after the concentration of the purified citrulline process liquid is performed by cooling to a temperature in the range of 5°C to about 45°C.

5. The method according to claim 1, wherein the Step 2 further comprises:
Step 2-1 of introducing the fermentation broth into a plurality of resin columns to separate the L-citrulline from impurities; and
Step 2-2 of introducing water into the resin columns in which L-citrulline has been retained, to obtain the purified citrulline process liquid comprising L-citrulline and water.

6. The method according to claim 5, wherein the resin columns provide a strong acid cation exchange resin.

7. The method according to claim 1, further comprising, between Step 2 and Step 3, filtering and decolorizing the purified citrulline process liquid.
